# EUROPEAN PATENT APPLICATION

(11) **EP 3 756 476 A1**
(43) Date of publication of application: **30.12.2020**
(21) Application number: 19382530.4
(22) Date of filing: 24.06.2019
(51) Int. Cl.: A23L 33/105, A61K 31/19, A61K 31/56, A61Q 19/00

(54) **APPLE WASTE EXTRACTION PROCESS**

(71) Applicant: Bioacores, S.L., 33428 Llanera, Asturias (ES)
(72) Inventor: BAYOD JASANADA, Miguel Santos, E-33428 Llanera, Asturias (ES); VALDÉS GÓMEZ, Alfonso Carlos, E-33428 Llanera, Asturias (ES); BALLESTEROS TOLOSANA, Alberto, E-33428 Llanera, Asturias (ES)
(74) Representative: ABG Intellectual Property Law, S.L.

(57) **Abstract**

The present invention refers to a process for the extraction of a product comprising ursolic acid and its use as food additive or food supplement, as cosmetic ingredient and as a medicament.

## Description

### FIELD OF THE INVENTION

The present invention relates to the field of recycling of apple waste, in particular to an apple waste extraction process to obtain ursolic acid.

### BACKGROUND

The apple production industry is growing, apple trees are cultivated worldwide and are the most widely grown species in the genus Malus. According to the FAO, total world apple production for 2017 was 83,139,326 metric tonnes. Part of this apple production is used for the production of food and drink products like apple slices or chips, apple juice, apple puree, cider, apple vinegar and distillates like calvados. The processing of these products generates apple peel and/or a solid residue after milling and pressing, known as apple pomace. Apple pomace can represent about 30% of the original fruit and contains peel, flesh, stem, core, seeds and juice residues. Fresh apple pomace contains large amounts of water.
Apple pomace is a multipurpose product with uses including fuel (ethanol production), direct burning, gasification, anaerobic digestion (methane generation), food (pomace jam, sauce, confectionery products such as pomace powder for toffees), pectin production, citric acid production, fibre extraction, and livestock feed. However, its production is a problem for manufacturers who have to manage large volumes of pomace generated daily.

The extraction of apple by-products to obtain bioactive compounds such as ursolic acid is known in the art. Ursolic acid is a bioactive triterpenoid widely found in fruits, as well as in herbs and spices like rosemary and thyme. Its chemical formula is:

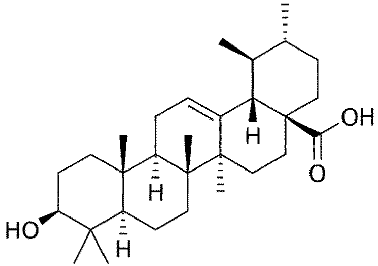

Ursolic acid has valuable biological properties like increasing the synthesis of elastin and inhibiting the activity of collagenase, both properties useful in the cosmetic industry. *In vitro,* ursolic acid inhibits the proliferation of various cancer cell types by inhibiting the STAT3 activation pathway and may also decrease proliferation of cancer cells and induce apoptosis. Ursolic acid inhibit JNK expression and IL-2 activation of JURKAT leukemic T Cells leading to the reduction in proliferation and T cell activation. ursolic acid also induces neural regeneration in mice after sciatic nerve injury. Therefore, it is a promising candidate for the development of new therapeutic approaches and for the prevention and treatment of several disease.

The extraction ursolic acid from apple by-products, in particular from apple peel, is known in the art. For example, CN103204895 discloses the use of a solution comprising water and a low chain alcohol in an extraction process from apple peel. CN106589044 describes the separation of ursolic acid from apple peel by an alkali extraction method using an alkali containing low carbon-chain alcohol or acetone aqueous solution.

The extraction of valuable by-products from apple pomace is also known. For example, C. Grigoras et al., (Industrial Crops and Products, 49 (2013) 794-804) describes the extraction of a mixture of compounds comprising ursolic acid from apple pomace. In particular, it discloses a microwave assisted extraction using three different extractant i.e. a) a 90:10 H₂O:MeOH mixture, b) ethanol (EtOH) and c) ethyl acetate (EtOAc). Polar compounds such as saccharides are generally avoided in the extraction of apple by-products. C. Grigoras et al. describes that the use of a water alcohol mixture (such as a H₂O:MeOH mixture) extracts the most polar compounds, ethanol extracts a mixture of polar and non-polar compounds and EtOAc extracts the major amount of phenolic compounds. C. Grigoras et al. states that the extraction solvent has a great influence on the composition of the extract. However, it does not study the effect of said extraction in the yield or purity of ursolic acid.

Grigoras et al. Journal of Engineering Studies and Research, vol. 18 (2012), no 1 pages 96-103 describes the extraction of bioactive compounds from pomace of four apple varieties. When comparing the different extraction methods, they state that the conventional methods for extracting natural compounds are mainly maceration and Soxhlet extraction. In spite of their efficiency these processes usually need long time and consume important volumes of solvent, therefore they suggest alternative extraction techniques. To assess the influence of the extraction process, they use microwave assisted extraction with solvents such as water/methanol mixture, pure ethanol and pure ethyl acetate.

In addition, patent publication KR20150130639 describes the use of method for the extraction of ursolic acid from apple pomace using sequentially hot water and 95% alcohol. The hot water extraction step removes the water-soluble substances (sugar component) and dietary fiber.

KR101393438 discloses a process for extracting ursolic acid from apple peel using a solvent containing acetic acid, preferably a mixture of ethanol and acetic acid.

In the prior art, great purification and separation effort is needed to remove unwanted components from apple waste, in order to obtain a product comprising ursolic acid with high yield and purity. Therefore, there is a clear need for new industrially scalable methods of extracting, purifying or isolating ursolic acid with high yield, low impurities and reduced costs.

### BRIEF DESCRIPTION OF THE INVENTION

The inventors have developed a new process for the extraction, isolation or purification of ursolic acid from apple waste, such as apple pomace and apple peel.

The inventors have observed that the yield of ursolic acid and its purity is insufficient with the extraction processes of the art. In particular, the inventors have found that using an extractant comprising a mixture of C₁-C₆ alkylcarboxylic acid and ethyl acetate, the process of the present invention significantly improves the yield and purity of the ursolic acid. Moreover, the inventors have observed that said extractant allows the separation of ursolic acid from complex by-products such as pectin, saccharides and others. Thus, the extraction step of the presented invention allows for an efficient separation of ursolic acid and reduces the presence of impurities in the final products of the process. In addition, since the process of the present invention is simple and inexpensive, it is suitable for the large-scale purification and/or production of ursolic acid.

Therefore, in a first aspect of the invention is directed to a process comprising:
i) providing apple waste;
ii) subjecting the apple waste of step (i) to an extraction to yield a liquid mixture comprising ursolic acid and a solid by-product; and
iii) subjecting the liquid mixture of step (ii) to one or more separation and/or purification steps to yield a product comprising ursolic acid;
wherein the extractant used in the extraction step ii) comprises an C₁-C₆ alkylcarboxylic acid or mixtures thereof and ethyl acetate.

A second aspect of the invention is directed to a product comprising ursolic acid obtained by the process of the present invention.

A further aspect of the present invention is directed to the use of the product comprising ursolic acid as a food additive, as a cosmetic ingredient or for therapy.

### FIGURES

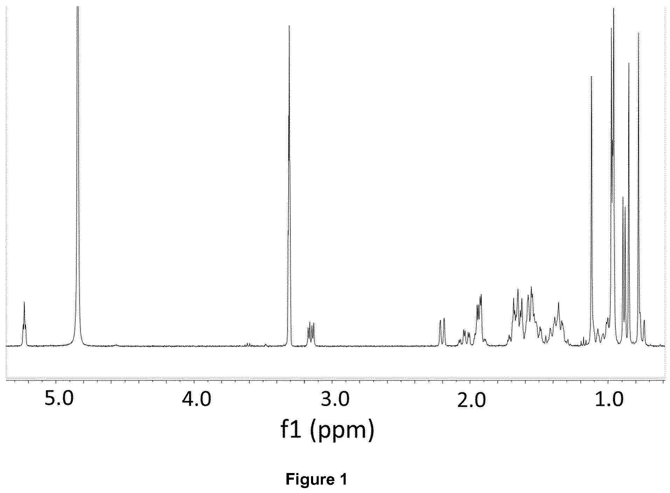

Figure 1 shows a proton nuclear magnetic resonance (HNMR) graph of the ursolic acid compound.

### DETAILED DESCRIPTION OF THE INVENTION

Unless defined otherwise, all technical and scientific terms used herein have the same meaning as commonly understood to one of ordinary skill in the art to which this disclosure belongs.

### Process

### Ursolic acid extraction

As defined above, in a first aspect, the present invention refers to a process, comprising:
i) providing apple waste;
ii) subjecting the apple waste of step (i) to an extraction to yield a liquid mixture comprising ursolic acid and a solid byproduct; and
iii) subjecting the liquid mixture of step (ii) to one or more separation and/or purification steps to yield a product comprising ursolic acid;
wherein the extractant used in the extraction step ii) comprises C₁-C₆ alkylcarboxylic acid or mixtures thereof and ethyl acetate.

In the context of the present invention, the term "apple waste" is understood as the waste solid product obtainable from the process of processing apple fruits in the food industry, for example the solid residue that remains after milling and/or pressing of apples for the production of cider, apple juice or puree, or from other similar methods. In a particular embodiment, the apple waste refers to apple pomace and/or apple peel. The apple waste generally comprises ursolic acid, pectin, antioxidants and saccharides among other components.

In the process of the invention, the starting material can be apple pomace, apple peel, or mixtures of these two waste products.

The apple waste can be in different grades of fermentation. It can also be in wet or dried form. Preferably, it is in dried solid form, with a content of water of less than 15 %. this reduces the volume and weight of the starting material to be handled and concentrates the components to be extracted. In a preferred embodiment, it is in the form of dried flakes or pellets; preferably flakes.

### Extraction

The process of the present invention comprises a step (ii) of subjecting the apple waste to an extraction to yield a liquid mixture comprising ursolic acid and a solid by-product. The inventors have found that an extraction with a solvent mixture comprising a C₁-C₆ alkylcarboxylic acid (or mixtures thereof) an ethyl acetate provides a good yield of ursolic acid, substantially removing pectin, other sacharides and phenolic and polyphenolic antioxidants, which can be recovered in further steps from the solid by-product.

In a particular embodiment, the C₁-C₆ alkylcarboxylic acid of the present invention is a C₁-C₅ alkylcarboxylic acid; preferably a C₁-C₄ alkylcarboxylic acid; more preferably formic, acetic or propionic acid. The most preferred acid is acetic acid. Mixtures of these acids can also be used.

In a particular embodiment, the extractant substantially consists of a mixture of C₁-C₆ alkylcarboxylic acid and ethyl acetate, with no other solvent present. Preferably, it is a mixture of acetic acid and ethyl acetate.

In a particular embodiment, the extractant comprises a mixture of C₁-C₆ alkylcarboxylic acid and ethyl acetate, wherein the C₁-C₆ alkylcarboxylic acid is in a volume percentage per volume of solvent of between 0.1 and 10; preferably between 1 and 8; more preferably between 2 and 6; even more preferably between 3 and 6, most preferably about 5.

In a particular embodiment, the extractant consist of a mixture of C₁-C₆ alkylcarboxylic acid as 1-10% in volume and ethyl acetate as 90-99% in volume; preferably of C₁-C₆ alkylcarboxylic acid as 3-7% in volume and ethyl acetate as 93-97% in volume.

In the context of the present invention, the expression "extraction" refers to the separation of a substance or a mixture of substances from the solid matrix (in this case apple waste, apple pomace or apple peels) using an extractant (i.e. a solvent or a solvent mixture).

The extraction of step (ii) in the process of the present invention can be done by any of the extraction methods known in the art. Preferably, extraction of step (ii) of the process of the present invention is selected from a pressurized fluid extraction, an ultrasound assisted extraction, an extraction under reflux and a microwave assisted extraction; preferably an extraction under reflux.

The extraction of step (ii) may be performed at a temperature between 1 and 300°C; preferably between 20 and 100°C; more preferably between 50 and 80°C.

The extraction of step (ii) may be performed for a period between 1 and 100 min; preferably between 10 and 80 min; more preferably between 20 and 70 min.

The extraction of step (ii) may be a single-stage or a multi-stage extraction.

### Step (iii)

The process of the present invention further comprises a step (iii) of subjecting the liquid mixture of step (ii) to one or more separation and/or purification steps to yield a product comprising ursolic acid.

In the context of the present invention, the expression "ursolic acid" is understood as known in the art (i.e. 3-hydroxy-urs-12-en-28-oic-acid or 3-hydroxy-12-ursen-28-ic acid compound) or its salts. The compound ursolic acid may comprise impurities.

### Solid-liquid separation

In a particular embodiment, the one or more separation and/or purification of step (iii) comprise:
A) a solid-liquid separation step to yield a liquid phase comprising ursolic acid and a solid byproduct.

In a particular embodiment, the solid-liquid separation is performed by filtration, sedimentation, centrifugation or a combination thereof; preferably by filtration. The filtration may be performed by any methods known in the art. The expert in the art may adapt the solid-liquid separation conditions to adequate them to the present invention.

In a particular embodiment, the solid-liquid separation is performed at a temperature below 50 °C, preferably below 40 °C, more preferably below 30°C.

In a particular embodiment, the liquid phase of the solid-liquid separation step (A) is washed. Preferably it is washed with water.

In another particular embodiment, the liquid phase of the solid-liquid separation step (A) is concentrated.

In a particular embodiment, the liquid phase of the solid-liquid separation step (A) is washed and concentrated.

In a particular embodiment, the liquid phase of the solid-liquid separation step (A) comprises ursolic acid and phenolic and/or polyphenolic antioxidants.

### Precipitation-separation step

In a particular embodiment, step (iii) of the process of the present invention comprises a precipitation-separation step to yield a product comprising ursolic acid.

In a particular embodiment, step (iii) further comprises a step B) of subjecting the liquid phase comprising ursolic acid of step (A) to one or more precipitation-separation steps to yield a solid phase comprising ursolic acid and a liquid phase; preferably a liquid phase comprising antioxidants.

In a more particular embodiment, step (iii) of the process of the present invention comprises a step (B) of subjecting the liquid phase comprising ursolic acid of step (A) to two precipitation-separation steps to yield a solid phase comprising ursolic acid and a liquid phase comprising antioxidants.

In a particular embodiment, the precipitation-separation steps are precipitation-filtration steps.

In a particular embodiment, the precipitation-separation step comprises:
i. a precipitation step comprising the addition of a solvent or a solvent mixture to yield a solid phase comprising ursolic acid and a liquid phase;
ii. a solid-liquid separation step to yield a solid phase comprising ursolic acid and a liquid phase.

In a more particular embodiment, the solvent or solvent mixture of step i. of the precipitation-separation step is a solvent mixture; preferably an azeotrope; more preferably an isopropanol/water azeotrope.

In a more particular embodiment, the solvent or solvent mixture of step i. of the precipitation-separation step is an organic solvent; preferably an organic solvent selected from an linear alkane or cycloalkane; preferably from a linear alkane; more preferably from a C₁-C₁₀ alkane; even more preferably from a C₂-C₈ alkane; more preferably it is hexane.

In a more particular embodiment, the solvent or solvent mixture of step i. of the precipitation-separation step is a mixture of hexane/methanol.

In a particular embodiment, the solid-liquid separation of step ii. is performed by filtration, sedimentation, centrifugation or a combination thereof; preferably by filtration. The filtration may be performed by any methods known in the art. The expert in the art may adapt the solid-liquid conditions to adequate them to the present invention.

In a more particular embodiment, the precipitation-separation step comprises a mixing step. The mixing step may be performed by any technique known in the art.

In a more particular embodiment, the precipitation-separation step comprises a reduction of temperature; preferably below 20°C; more preferably below 15°C; even more preferably below 12°C; even much more preferably around 10°C.

In a preferred embodiment, the precipitation-separation step comprises:
i. a precipitation step comprising the addition of hexane, mixing and a temperature reduction to around 10°C to yield a solid phase comprising ursolic acid and a liquid phase; and
ii. a solid-liquid separation step to yield a solid phase comprising ursolic acid and a liquid phase; preferably a liquid organic phase comprising antioxidants.

In a more preferred embodiment, the precipitation-separation step comprises:
i. a precipitation step comprising the addition of isopropanol or an isopropanol/water mixture, and mixing to yield a solid phase comprising ursolic acid and a liquid phase; and
ii. a solid-liquid separation step to yield a solid phase comprising ursolic acid and a liquid phase; preferably a liquid phase comprising antioxidants.

In a more particular step, the precipitation-separation step of the present invention comprises a precipitation-separation step comprising the addition of isopropanol or isopropanol/water and another precipitation-separation step comprising the addition of hexane.

In another more particular step, the precipitation-separation step of the present invention comprises an extraction-separation step; preferably, an extraction-filtration step comprising the addition of isopropanol or isopropanol/water and a precipitation-separation step comprising the addition of hexane.

In another more particular step, the precipitation-separation step of the present invention comprises an extraction-separation step; preferably, a precipitation-separation step comprising the addition of isopropanol or isopropanol/water and an extraction-separation step comprising the addition of hexane.

In a particular embodiment, the precipitation-separation step is a single or multi step.

In a more particular embodiment, the precipitation-separation step includes a recrystallization step.

In a more particular embodiment, step (iii) of the process of the present invention comprises a step (B) of
subjecting the liquid phase comprising ursolic acid of step (A) to two precipitation-separation steps to yield a solid phase comprising ursolic acid and a liquid phase comprising antioxidants;
wherein isopropanol or an azeotrope of isopropanol/water mixture is added in the first precipitation separation step; and
wherein hexane is added in the second precipitation-separation step.

In a more particular embodiment, step (iii) of the process of the present invention comprises a step (B) of
subjecting the liquid phase comprising ursolic acid of step (A) to a washing step followed by two precipitation-separation steps to yield a solid phase comprising ursolic acid and a liquid phase comprising antioxidants;
wherein isopropanol or an azeotrope of isopropanol/water mixture is added in the first precipitation separation step; and
wherein hexane is added in the second precipitation separation step.

In a more particular embodiment, step (iii) of the process of the present invention comprises a step (B) of
- subjecting the liquid phase comprising ursolic acid of step (A) to a precipitation-separation step with isopropanol or an azeotrope of isopropanol/water to yield a solid phase comprising ursolic acid and a liquid phase, and
- subjecting the solid phase to an extraction step with hexane.

In a more particular embodiment, step (iii) of the process of the present invention comprises a step (B) of
- subjecting the liquid phase comprising ursolic acid of step (A) to a washing step followed by a precipitation-separation step with isopropanol or an azeotrope of isopropanol/water to yield a solid phase comprising ursolic acid and a liquid phase, and
- subjecting the solid phase to an extraction step with hexane.

In a particular embodiment, the liquid phase comprising ursolic acid of step (A) is subjected to a washing step.

Without being bound by a particular theory, it is believed that the precipitation-separation step of the present invention allows the separation of a product comprising ursolic acid from a product comprising antioxidants; preferably of a solid comprising ursolic acid. A high yield of ursolic acid and a reduced amount of antioxidants such as tannic acid, chlorogenic acid, phlorizin and quercetin has been observed in the solid comprising ursolic acid.

### Purification steps

In a particular embodiment, the process of the present invention comprises subjecting any of the liquid or solid phases or products obtained in the any of the previous steps to one or more purification steps.

In a more particular embodiment, the at least one further purification is selected from membrane separation, filtration, evaporation, extraction, distillation, recrystallization and/or a combination thereof. Preferably the at least one further purification comprises a distillation; more preferably comprises a membrane separation followed by a distillation. In view of the composition of the liquid phase comprising ursolic acid obtained in any of the previous steps, the skilled person can devise a further purification scheme in order to obtain ursolic acid with a higher degree of purity.

### Evaporation

In a more particular embodiment, any of the liquid phases obtained in any of the steps of the process of the present invention is subjected to one or more evaporation steps; preferably partial evaporation steps; more preferably partial evaporation steps under vacuum.

### Recirculation

In an even more particular embodiment, any of the liquid phases obtained in any of the steps of the process of the present invention is subjected to one or more recirculation step.

In a particular embodiment, the evaporation step of the process of the present invention is performed in a unit selected form thin-film evaporator, wiped film evaporator, falling film evaporator, forcer circulator evaporator, scrapped surface evaporator and agitated thin film evaporator, preferably a thin-film evaporator. In more a particular embodiment, the evaporation step of the process of the present invention is performed at between 1 and 160°C and at a pressure of between 1-200 mbars.

In a particular embodiment, the evaporation step of the process of the present invention yields a concentrated liquid phase; preferably, a concentrated liquid phase concentrated to below a 50 wt% of the weight of the original liquid phase; more preferably below a 20 wt%; even more preferably to an around 10 wt%.

In an even more particular embodiment, the evaporation step of the process leads to an solvent vapor (i.e. an organic vapor or an aqueous vapor); wherein said solvent vapor is optionally be condensed and reused in other steps of the present invention. The optional the evaporation step of the process leads to a reduction of the total amount of organic solvent used in the whole process since the vapors produced during the evaporation may be recycled. Additionally, by using an optional evaporation step, the global efficiency of the process is increased.

### Washing steps

In a more particular embodiment, any of the liquid phases obtained in any of the steps of the process of the present invention is subjected to one or more washing steps; preferably washing step comprising a washing solution; more preferably an aqueous washing solution.

In a preferred embodiment, the liquid phase comprising ursolic acid of step (iii) of the present invention is subjected to a washing step; preferably washing step comprising a washing solution; more preferably an aqueous washing solution; more preferably water.

### Ursolic acid

A second aspect of the invention is directed to a product comprising ursolic acid obtainable by the process of the present invention in any of its embodiments.

In a particular embodiment, the product comprising ursolic acid of the present invention has a purity percentage over 8%, preferably over 10%, more preferably over 40%; even more preferably over 50%; even more preferably 80%, even more preferably 90% and over 95%, even more preferably over 98%, even more preferably over 99% .

In a particular embodiment, the product comprising ursolic acid has a purity percentage of between 50 and 60%.

The product comprising ursolic acid may comprise impurities.

In the context of the present invention, the Folin-Ciocalteu Index has been calculated as mg of tannic acid per g of sample by using a colorimetric in vitro assay using the Folin-Ciocalteu reagent (mixture of phosphomolybdate and phosphotungstate) also named the Gallic acid equivalence method (GAE) as known in the art.

In a particular embodiment, the product comprising ursolic acid of the present invention does not comprise a significant amount of chlorogenic acid.

In a particular embodiment, the product comprising ursolic acid of the present invention does not comprise a significant amount of quercetin.

In a particular embodiment, the product comprising ursolic acid of the present invention comprises less than 1 mg of phlorizin per 1 g of sample.

In the context of the present invention the term "antioxidant" refers to compounds that are capable of inhibiting reactions promoted by oxygen, thus avoiding oxidation and rancidity of the compositions. Non-limitative examples of antioxidants are phenolic and triterpenic compounds. Non-limiting examples of phenolic compounds are chlorogenic acid, tannic acid, gallid acid (GA), p-coumaric acid (pCA), catechin (CAT), phloridzin (PH), quercetin (QUE) and rutin (RU). Non-limiting examples of triterpenic compounds are betulinic, oleanolic acid (OA), erythrodiol, and uvaol (Uv).

In a particular embodiment, the antioxidants are chlorogenic acid, tannic acid, gallid acid (GA), p-coumaric acid (pCA), catechin (CAT), phloridzin (PH), quercetin (QUE) and rutin (RU), betulinic, oleanolic acid (OA), erythrodiol, and uvaol (Uv); preferably from chlorogenic acid, tannic acid, phloridzin (PH) and quercetin (QUE).

### Use

A further aspect of the present invention is directed to the use of the product comprising ursolic acid of the present invention in any of its particular embodiments as food additive, food supplement, or as a cosmetic ingredient.

In another aspect, the invention is also directed to the ursolic acid obtained by the above described processes for use in a medicament, preferably in the treatment of cancer, neurogeneratives diseases or for neuron regeneration.

### EXAMPLES

The invention is illustrated by means of the following examples, which in no case limit the scope of the invention.

### Example 1 Ursolic acid extraction

210 grams of dried apple waste (apple pomace and apple peel in dried form obtained from Apple (*Malus Domestica*)) were extracted under reflux with 500 ml of a mixed solvent comprising 95 vol. % of ethyl acetate (AcOEt) and 5 vol. % of acetic acid (AcOH) for 30-60 minutes at a temperature of 50-77 °C.

After cooling to a temperature below 30°C, the suspension was filtered and a liquid extract comprising ursolic acid was separated from a solid by-product.

The obtained extract (organic phase) was washed with 250 ml demineralized water. A reduction in aqueous soluble residues is observed.

The washed extract (organic phase) was subsequently concentrated under vacuum to 10 % of its original weight.

The washed and concentrated extract (organic phase) was further washed using 100 ml of demineralized water.

The obtained extract (organic phase) was further concentrated to remove the remaining ethyl acetate.

Upon addition of 50 ml n-hexane, the mixture (i.e. organic phase) was stirred and cooled to about 10 °C to precipitate a solid mixture. The obtained solid mixture was filtered off and dried. The yield of the extraction was 2,1 gram of solid ursolic acid with a purity of over 50 % (between a 50-60%), as determined by high performance liquid chromatography (HPLC) (Chromatograph waters E2695 Separations Module and 2996 Photodiode Array Detector and a nucleosil C18 column, using as a mobile phase methanol and water in a ratio 85:15 and detected at 201 nm). Thus, by the process of the present invention 1050 mg of ursolic acid have been extracted from 210 grams of dried apple pulp (5 mg per gram).

The results of the HPLC analysis of the final product are shown in Table 1. Results show that an insignificant amount of polyphenols, chlorogenic acid, phlorizin and querceting are found in the ursolic acid extract obtained by the process described above.

In addition, Figure 1 shows the proton nuclear magnetic resonance (HNMR) results for ursolic acid (recorder on a Brucker Av-400; internal standard 4-bromobenzaldehyde).

**Table1.**

| Compound | Analysis |
|---|---|
| Ursolic acid (HPLC) | 50-60% |
| Folin & Ciocalteu Index¹ | 17.2 |
| Chlorogenic acid² | nd³ |
| Phlorizin² | < 0.6 |
| Quercetin² | nd³ |

| | |
|---|---|
| *¹total polyphenols (mg tannic acid*/*g sample)* *²mg*/*g sample* *³not detected* | |

### Example 2 Ursolic acid extraction from apple waste

A process similar to the one described in Example 1, but without the final extraction step with hexane. It was performed on dried apple waste and the results obtained are shown in Table 2 below. A residual amount of chlorogenic acid, phlorizin and quercetin are found in the extract obtained by this process in comparison with those obtained from in example 1. In addition, the ursolic acid obtained is significantly less. Moreover, the Folin & Ciocalteu, for the determination of phenolic and polyphenolic antioxidants index is significantly smaller.

**Table 2**

| Compound | Analysis |
|---|---|
| Ursolic acid (HPLC) | 8-10% |
| Folin & Ciocalteu Index¹ | 7.2 |
| Chlorogenic acid² | 0.1 |
| Phlorizin² | 0.9 |
| Quercetin² | 1.1 |

| | |
|---|---|
| *¹total polyphenols (mg tannic acid*/*g sample)* *²mg*/*g sample* *³not detected* | |

## Claims

1. A process comprising:
i) providing apple waste;
ii) subjecting the apple waste of step (i) to an extraction to yield a liquid mixture comprising ursolic acid and a solid by-product; and
iii) subjecting the liquid mixture of step (ii) to one or more separation and/or purification steps to yield a product comprising ursolic acid;
**characterized in that** the extractant used in the extraction step ii) comprises a C₁-C₆ alkylcarboxylic acid or mixtures thereof and ethyl acetate.

2. The process according to claim 1, wherein the extractant used in the extraction step ii) consists of a mixture of C₁-C₆ alkylcarboxylic acid or mixtures thereof and ethyl acetate.

3. The process according to claim 1 or 2, wherein the C₁-C₆ alkylcarboxylic acid or mixtures thereof is in a volume percentage of between 0.1 and 10 % in volume of the extractant.

4. The process according to any of the previous claims, wherein the extractant is a mixture of 3-7% in volume of C₁-C₆ alkylcarboxylic acid or mixtures thereof, and 93-97% in volume of ethyl acetate.

5. The process according to any of the previous claims wherein the extraction is performed under reflux.

6. The process according to any of the previous claims wherein the C₁-C₆ alkylcarboxylic acid is selected from formic, acetic or propionic, preferably acetic acid.

7. The process according to any of the previous claims wherein step (iii) comprises a precipitation-separation step with an organic apolar solvent to yield a solid phase comprising ursolic acid and a liquid product comprising antioxidants.

8. The process according to claim 7, wherein the organic apolar solvent is an hydrocarbon, preferably hexane.

9. A product comprising ursolic acid obtained by the process according to any of claims 1-8.

10. The product comprising ursolic acid according to claim 9 wherein the product comprising ursolic acid of the present invention has a purity percentage over 50%, more preferably over 90%, even more preferably over 95 %.

11. Use of the product comprising ursolic acid according to claims 9 or 10 as a food additive or food supplement.

12. Use of the product comprising ursolic acid according to claims 9 or 10 as a cosmetic ingredient.

13. Ursolic acid according to claims 9 or 10 for use as a medicament.
